(19) Europäisches Patentamt
European Patent Office
Office européen des brevets

(11) EP 2 497 535 B1

(12) EUROPEAN PATENT SPECIFICATION

(45) Date of publication and mention
of the grant of the patent:
**07.05.2014 Bulletin 2014/19**

(51) Int Cl.:
***A61N 5/10*** (2006.01)

(21) Application number: **11157125.3**

(22) Date of filing: **07.03.2011**

(54) **Water equivalent depth measurement**

Wasseräquivalent-Tiefenmessung

Mesure de la profondeur de l'équivalent d'eau

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.09.2012 Bulletin 2012/37**

(73) Proprietor: **Ion Beam Applications**
**1348 Louvain-la-Neuve (BE)**

(72) Inventors:
• **Gottschalk, Bernard**
**Cambridge, MA 02138 - 1603 (US)**

• **Jongen, Yves**
**1348, Louvain-la-Neuve (BE)**

(74) Representative: **Denis, Samuel et al**
**Pecher & de Groote sprl**
**Centre Monnet**
**Avenue Jean Monnet, 1**
**1348 Louvain-la-Neuve (BE)**

(56) References cited:
• **HSIAO-MING LU: "A potential method for in vivo
range verification in proton therapy treatment",
PHYS. MED. BIOL., vol. 53, no. 5, 19 February 2008
(2008-02-19), pages 1413-1424, XP002633969,**

## Description

### Field of the invention

The invention relates to the field of charged particle therapy. More specifically, the invention relates to a method as defined in claim 1 for determining a water equivalent depth between an entrance point and a reference point, said entrance point lying on an external surface of a body, said reference point being a point at which a charged particle beam detector is placed, said method comprising the steps of :

a) providing means for delivering a charged particle beam to said body in a beam direction defined from said entrance point to said reference point;
b) choosing a maximum energy value such that a charged particle beam having said maximum energy value and being sent in said beam direction penetrates the body from said entrance point up to at least said reference point;
c) choosing a minimum energy value such that a charged particle beam having said minimum energy value and being sent in said beam direction penetrates the body from said entrance point and is stopped in said body before reaching said reference point;
d) sending in said beam direction a charged particle beam that is time modulated in energy between said minimum energy value and said maximum energy value;
e) acquiring a time dependent response of the charged particle beam detector.

The invention also relates to a device as defined in claim 10 for determining a water equivalent depth between an entrance point and a reference point, said entrance point lying on an external surface of a body, said device comprising :

- a charged particle beam detector that is placed at the reference point;
- means for acquiring a time dependent response of said charged particle beam detector.

The invention also relates to a program as defined in claim 14 for determining a water equivalent depth between an entrance point and a reference point, said entrance point lying on an external surface of a body, said reference point being a point at which a charged particle beam detector is placed, said program comprising :

- a software module for acquiring a time dependent response of said charged particle beam detector.

### Description of prior art

An interesting property of charged particle therapy such as proton therapy is the sharp fall-off of the dose to depth distribution induced by a charged particle beam traversing a body. This distribution is, however, very sensitive to variations of the water equivalent depth (WED) in tissues along the beam direction of the charged particle beam. This leads to uncertainties in treatment planning. For this reason, a direct verification of the WED of a charged particle beam in a body is desirable.

In the document "A potential method for in vivo range verification in proton therapy treatment" by Hsiao-Ming Lu published in Phys. Med. Biol. 53 (2008) 1413-1424, the time dependent response of a charged particle beam detector such as an ionization chamber exposed to a time-dependent energy modulated proton beam was studied as a function of the depth of the charged particle beam detector in a water tank.

To produce a time-dependent energy modulated proton beam, one can use for instance a modulator wheel as it is explained in the Hsiao-Ming Lu's publication. Such a wheel typically contains different segments of absorbing materials with various thicknesses. As the modulator wheel rotates, typically at a constant speed of 600 rotations per minute, the charged particle beam passes through one segment at a time. As a consequence, a charged particle beam that is time modulated between a minimum and maximum energy value is induced at the exit of the modulator wheel.

From the results of the survey published in Phys. Med. Biol. 53 (2008) 1413-1424, it was shown that a charged particle beam detector exposed to a charged particle beam that is time modulated in energy presents time dependent patterns that are characteristic of the depth at which the charged particle beam detector is placed in a water tank. So, one can use these patterns as a unique coding of the water equivalent depths (WEDs). The collection of the patterns at different depths of the charged particle beam detector can be viewed as forming a ruler, where each mark corresponds to a unique pattern. This ruler can be obtained during a calibration phase by measurement or by calculation of the time dependent response of a charged particle beam detector that is placed (or assumed to be placed) at different depths in a phantom such as a water tank. After, by positioning the charged particle beam detector used in the calibration phase at a point that we name reference point (this reference point can lie in a body), one can deduce the WED corresponding to this reference point by matching the pattern measured by the charged particle beam detector at the reference point to one of the patterns determined in the calibration phase.

The article published in Phys. Med. Biol. 53 (2008), 1413-1424, proposes a simple method for this pattern matching that is minimizing the following least-square difference

$$L(x) = \int_0^T [\lambda f_m(t) - f_r(x,t)]^2 dt \qquad \text{(Eq. 1)}$$

with respect to depth x. The depth x corresponding to the minimum value of $L(x)$ is the sought WED. The function $f_r(x,t)$ represents the patterns determined during the calibration phase, and $f_m(t)$ is the measured time dependent response of the charged particle beam detector positioned at the WED to be determined. The function L(x) is also minimized with respect to a scale factor $\lambda$ because the pattern matching has to be purely based on the shape of the time dependence of the measured signal, independently of its absolute magnitude.

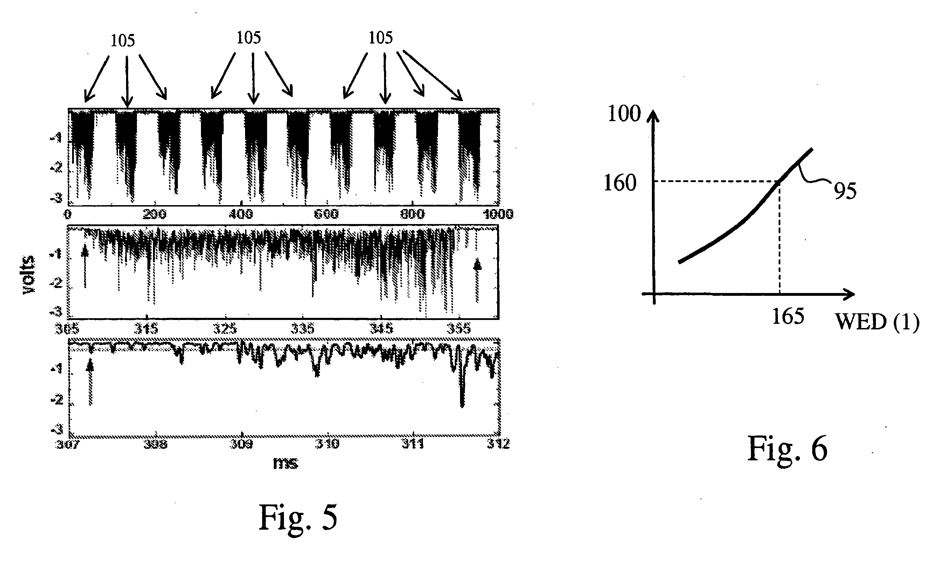

By using the method proposed by Hsiao-Ming Lu, one can hope to have a WED precision of about 1 mm in a homogeneous water phantom. A drawback of the method proposed by Hsiao-Ming Lu is the necessity to measure enough patterns at enough different depths during the calibration phase when a fine WED resolution is wanted, see for instance figure 5 of the publication from Phys. Med. Biol. 53 (2008) 1413-1424. This leads to a relatively long calibration phase when such a fine WED resolution is desired.

## Summary of the invention

According to a first aspect of the invention, it is an object to provide a method for determining the WED of a charged particle beam that requires a shorter calibration procedure. To this end, the method according to the invention is characterized in that the method further comprises the steps of

f) determining a value of a statistical parameter of said time dependent response;
g) providing a pre-determined calibration curve expressing a relationship between values of said statistical parameter and water equivalent depths;
h) determining from said calibration curve the water equivalent depth between the entrance point and the reference point corresponding to the value of the statistical parameter determined in step f).

The inventors have studied the values of different statistical parameters of time dependent responses of charged particle beam detectors when such detectors are subjected to a charged particle beam that is time modulated in energy. More precisely, they have studied the variation of such values when the charged particle beam detectors are positioned at different depths in a water tank. They have found that these values are smoothly and monotonically dependent of the depths of the charged particle beam detectors in the water tank. Thanks to this smooth dependence, one can readily finds a curve (for example by interpolation) that relates these values to the different depths. By identifying these different depths to WEDs, one then has a calibration curve relating values of one or of different statistical parameters to WEDs. Because of the smooth variation of the values of some statistical parameters to the different depths, only few points need to be determined in a calibration procedure. As a consequence, the method of the invention requires a shorter calibration phase than Lu's method. Once having this calibration curve, one can after determine the WED of a reference distance pointing to a position where a charged particle beam detector is placed by following the method of the invention.

The method of the invention presents other advantages. A value of a statistical parameter can be easily computed for either smooth or micro pulses (i.e. non-smooth) distributions. Such non-smooth distributions are typically obtained when the size of the charged particle beam detector is small, for instance when its size is equal or less than around 1 mm$^2$ x 2,5 $\mu$m (2,5 $\mu$m is the thickness of the charged particle beam detector along a beam direction of the charged particle beam). As a consequence, the use of a statistical parameter of the time dependent response of a charged particle beam detector presents better results than a shape matching method that uses equation (1) for determining WED. Moreover, the method according to the invention is less sensitive to noise with respect to Hsiao-Ming Lu's method.

It is a further object of the invention to provide a method for determining the WED of a charged particle beam with a higher precision. To this end, the method of the invention is preferably characterized in that said statistical parameter is a root mean square width of said time dependent response. With respect to Hsiao-Ming Lu's method, the method of the invention using a value of a root mean square width for determining the WED presents a higher accuracy. As shown in an example below, one can then obtain a precision of around 0,3 mm in WED determination, such a value being better than the precision of around 1 mm of the Hsiao-Ming Lu's method.

It is a further object of the invention to provide a method for determining the WED as a direct verification during or just before the actual treatment, for example in cases where the dose rate function is measured with a detector in a body cavity (e.g. oral cavity, oesophagus, rectum). To this end, the method of the invention is preferably characterized in that the reference point is positioned inside the body into which the particle beam travels. As the charged particle beam detector is positioned at the reference point, it can then consist in an implantable charged particle beam detector.

It is yet a further object of the invention to provide a method that does not need any insertion of a charged particle beam detector in a body for the determination of the WED of a charged particle beam traversing said body. To this end, the method of the invention is preferably characterized in that the reference point is positioned outside the body through which the charged particle beam travels. As the charged particle beam detector is positioned at the reference point, one then does not need to insert said charged particle beam detector into said body. The detector can for example be directly fixed to the patient or attached to a patient table.

It is yet another object of the invention to provide a method that uses a small, rugged and inexpensive charged particle beam detector. To this end, the charged particle beam detector preferably comprises a semiconductor diode for detecting the charged particle beam. Semiconductor diodes make good charged particle beam detectors that can be small, rugged and inexpensive.

It is yet another object of the invention to obtain the pre-determined calibration curve experimentally. To this end, the method of the invention is preferably characterized in that the pre-determined calibration curve is obtained from the following steps:

> i. positioning said charged particle beam detector at a given position in a water or water-equivalent calibration phantom;
> ii. sending to said charged particle beam detector the charged particle beam that is time modulated in energy between said minimum and said maximum energy value;
> iii. acquiring a time dependent response of said charged particle beam detector;
> iv. determining a value of said statistical parameter from said time dependent response;
> v. determining a water equivalent depth, said water equivalent depth being the distance between the entrance point of said charged particle beam in said calibration phantom and said given position;
> vi. repeating steps i. to v. n times, n being an integer larger than one;
> vii. establishing a calibration curve that relates the values of the statistical parameter determined in step iv. and the water equivalent depth determined in step v..

It is yet another object of the invention to provide a method that allows one to study the spatial variation of the WED in a body. To this end, the method of the invention is preferably characterized in that the charged particle beam detector comprises an array of semiconductor diodes placed transversally with respect to the charged particle beam. By acquiring the time dependent responses of each semiconductor diode of the array, one can deduce a value of a statistical parameter for each position of such diodes and determine the corresponding WEDs. By comparing these WEDs, one can have an evaluation of the spatial variation of the WED in a body.

According to another aspect of the invention, the inventors propose a device for determining a WED between an entrance point and a reference point, said entrance point lying on an external surface of a body, said device being characterized in that said it further comprises :

- means for determining a value of a statistical parameter of said time dependent response;
- means for loading a pre-determined calibration curve expressing a relationship between values of said statistical parameter and water equivalent depths;
- means for determining from said calibration curve the water equivalent depth corresponding to the value of the statistical parameter of the time dependent response of the charged particle beam detector placed at the reference point.

According to another aspect of the invention, the inventors propose a program for determining the WED between an entrance point and a reference point, said entrance point lying on an external surface of a body, said reference point being a point at which a charged particle beam detector is placed, said program being characterized in that said it further comprises :

- a software module for determining a value of a statistical parameter of said time dependent response;

- a software module for loading a pre-determined calibration curve expressing a relationship between values of said statistical parameter and water equivalent depths;

- a software module for determining from said calibration curve the water equivalent depth corresponding to the value of the statistical parameter of the time dependent response of the charged particle beam detector placed at the reference point.

## Short description of the drawings

These and further aspects of the invention will be explained in greater detail by way of example and with reference to the accompanying drawings in which:

Fig.1      shows a typical measurement setup used for the method of the invention;

Fig.2      shows an illustration of a Bragg peak;

Fig.3      shows an illustration of a modulator wheel;

Fig.4      shows time dependent dose rates calculated at twenty-one different depths in a water tank along a beam direction of a charged particle beam that is time modulated in energy;

Fig.5      shows the time dependent response of a PTW Model T60012 semiconductor diode that is placed in a water phantom and subjected to a charged particle beam that is time modulated in energy;

Fig.6      shows a schematic calibration curve;

Fig.7      shows a first example of experimental calibration curve;

Fig.8      shows a second example of experimental calibration curve;

Fig.9      shows a transimpedance amplifier according to the invention;

Fig.10     shows a device according to the invention;

Fig.11     shows, for various depths of a charged particle beam detector in a water tank, the difference between its actual depth and the depth estimated from the method of the invention.

The figures are not drawn to scale. Generally, identical components are denoted by the same reference numerals in the figures.

## Detailed description of preferred embodiments

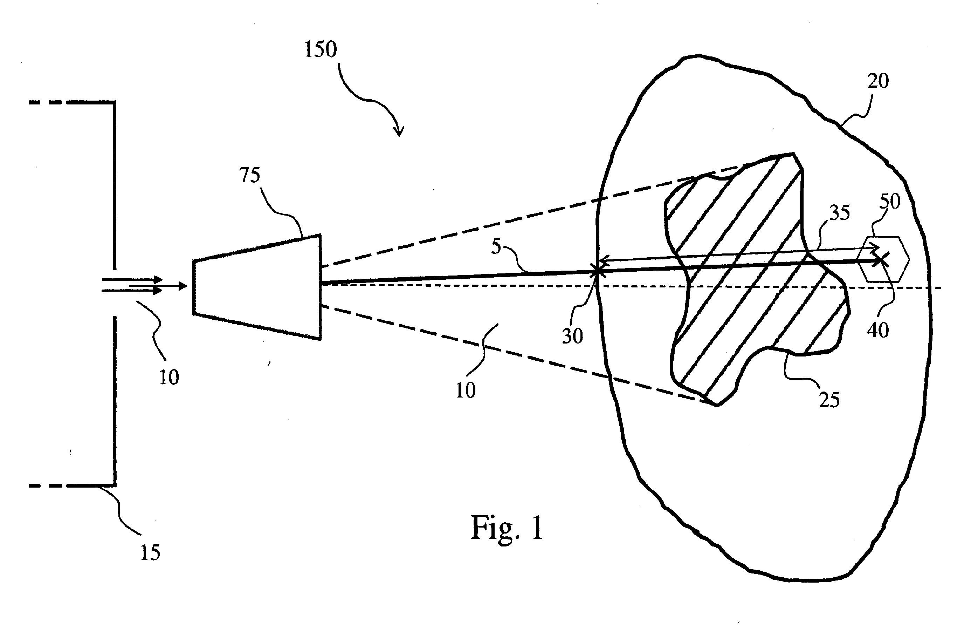

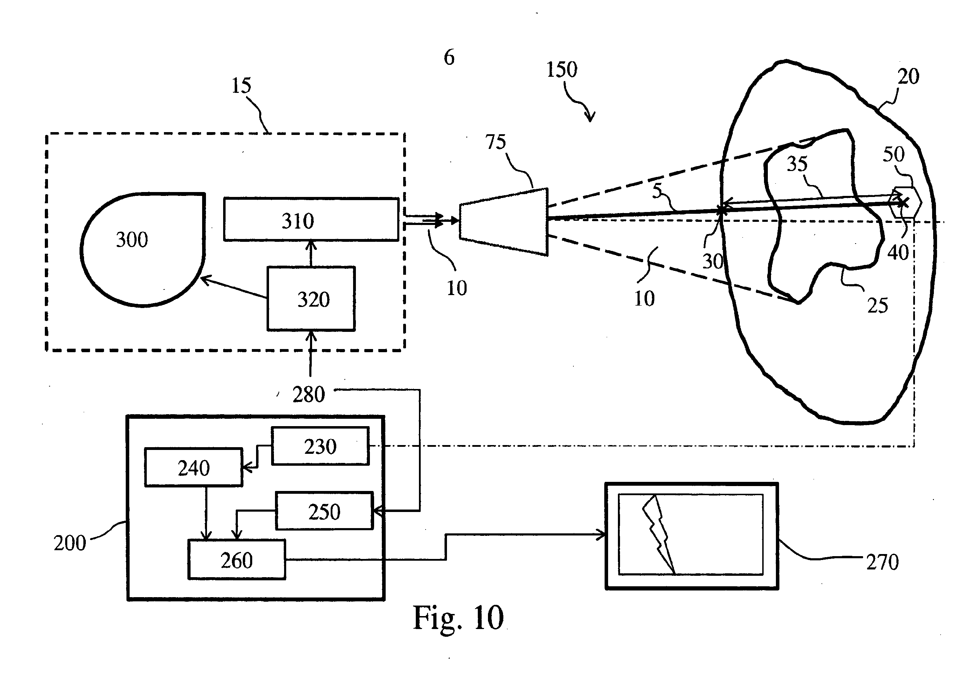

Charged particle therapy consists in sending to a target volume 25 a charged particle beam 10. The target volume 25 can be for instance a tumour. Figure 1 shows an example of a setup 150 that can be used for charged particle therapy. Means 15 send a charged particle beam 10, such as a proton beam, to a body 20 having a target volume 25 to be irradiated for treatment. On opposite to figure 1 that is not drawn to scale, the target volume 25 is generally much smaller than the body 20. In the same way, means 15 are typically much larger than the body 20. Means 15 are known by the one skilled in the art; as shown in figure 10, it typically includes a accelerator 300 of charged particles, such as a cyclotron, a beamline 310 to transport a charged particle beam 10 to the body 20 and a control system 320 that controls the accelerator 300 and the beamline 310. To irradiate the entire target volume 25, the charged particle beam 10 can be scattered thanks to the use of a nozzle 75 as shown in figure 1. As a result of the scattering process, the charged particle beam 10 at the exit of the nozzle 75 is wide and diverging. Another possibility to irradiate the entire target volume 25 is to use a magnetically scanned beam (typically, the scanning process takes place in the nozzle 75 thanks to the use of scanning magnets). In this case, the charged particle beam 10 penetrating the body 20 is more concentrate (and sometimes named pencil beam) but is typically scanned thanks to the use of varying magnetic fields that change the beam direction 5 of the charged particle beam 10. The scanning process allows one to irradiate the entire target volume 25.

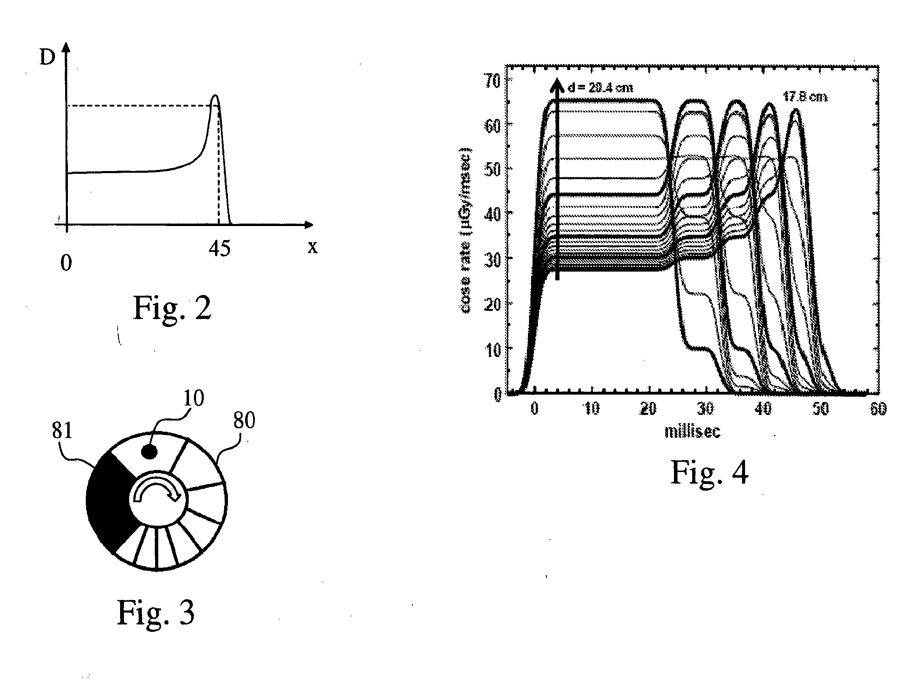

If one considers a charged particle beam 10 having a given energy, the variation of the dose that is induced in a body 20 at different depths along a beam direction 5 typically has the shape of figure 2. This figure shows an illustration of a Bragg peak (D stands for the dose, and x for the depth in the body 20). The zero value of the depth x corresponds to the entry of the charged particle beam 10 in the body 20. The one skilled in the art typically defines a depth 45 up to which the charged particle beam 10 penetrates. Beyond this depth 45, the one skilled in the art typically says that the charged particle beam 10 is stopped. This depth 45 is generally defined as the depth before which the dose falls below a given threshold after the peak, for example 90 % of the maximum dose induced in the body, which means 90 % of the dose value corresponding to the maximum of the Bragg peak. One can choose any criterion rather than 90 %.

In order to irradiate a target volume 25 along its entire depth, one need to use a charged particle beam 10 that presents different energy values. This leads to different depths 45 up to the charged particle beam 10 penetrates and so to different depths at which a maximum dose is delivered. One possibility to have a charged particle beam 10 that is modulated in energy is to send it through an energy modulator, for instance through a modulator wheel 80. When a scattered beam is used, such a modulator wheel 80 is typically inserted in the nozzle before scattering takes place. Figure 3 shows an illustration of a modulator wheel 80: it comprises a series of segments that have absorbing materials of different thicknesses. The black segment 81 of the modulator wheel 80 of figure 2 corresponds to a total absorption of an incident charged particle beam 10. A modulator wheel 80 typically spins at a constant speed, for instance 600 rotations per minute. The black point in figure 2 shows the place where the charged particle beam 10 crosses the modulator wheel 80 for a given angular position of the modulator wheel 80. As the modulator wheel 80 rotates, the charged particle beam 10 that points towards a given position passes through different segments having different absorbing properties. As a consequence, the charged particle beam 10 induces in the body 20 a series of Bragg peaks spread out in depth. By properly specifying the angular span of each segment of the range modulator, the superposition of these Bragg peaks form a flat region in the depth-dose distribution, i.e. a region having an uniform dose which is commonly referred as a spread-out Bragg peak (SOBP) by the one skilled in the art.

We now turn to detail the different steps of the method of the invention. The method of the invention aims at determining the water equivalent depth 1 between an entrance point 30 and a reference point 40. The entrance point 30 lies on an external surface of a body 20. Two examples for the body 20 are a phantom and a human body. A beam direction 5 can be defined from the entrance point 30 to the reference point 40. So, the reference point 40 lies downstream the entrance point 30 along such a beam direction 5. In figure 1, an example of entrance point 30 and reference point 40 is shown. The reference point 40 lies preferably inside the body 20 but the reference point 40 can also be located on an external surface of the body 20 or outside de body 20. When the reference point 40 is inside a body 20, it can be located in a natural cavity such as the rectum, or under the skin of a body 20. A charged particle beam detector 50 is placed at the reference point 40. Two examples of charged particle beam detector 50 are an ionization chamber and a semiconductor diode 110.

To carry out the method of the invention, one needs to provide means for delivering a charged particle beam 10 in a beam direction 5 as defined above. Such means typically include a source setup (or means 15) and a nozzle 75. The method of the invention works with system having a scattering or a scanning nozzle 75. In the latter case, the magnetic settings of the scanning magnets are set such that a pencil charged particle beam 10 points along the beam direction 5 defined above.

Then, one has to choose a maximum 65 and minimum 70 energy value. The maximum energy value 65 is such that a charged particle beam 10 having this maximum energy value 65 and being sent in the beam direction 5 defined above penetrates the body 20 from said entrance point 30 up to at least said reference point 40. The distance 45 up to a charged particle beam 10 penetrates has been defined above with figure 2. The minimum energy value 70 is such that a charged particle beam 10 having this minimum energy value 70 and being sent in the beam direction 5 defined above penetrates the body 20 from said entrance point 30 and is stopped in the body 20 before reaching the reference point 40. The distance 45 up to which a charged particle beam 10 penetrates in a body 20 has been defined above with figure 2. The way of determining such energy values are known by the one skilled in the art. Typically such values can be obtained from a treatment planning if one knows, at least approximately the absorption properties of the body 20 along the beam direction 5.

The next step of the method of the invention is to send in the beam direction 5 a charged particle beam 10 that is time modulated in energy between the minimum 70 and maximum 65 energy value defined above. This means that the energy of the charged particle beam 10 has to vary in time between a minimum 70 and a maximum 65 energy value. Preferably, the time variation of the energy of the charged particle beam 10 is a monotically decreasing function from the maximum 65 to the minimum 70 energy value. More preferably, the time variation of the energy of the charged particle beam 10 is a periodic function with respect to time. To modulate the energy of a charged particle beam 10, one can use a modulator wheel 80 as defined above with figure 2. Typically, such a modulator wheel 80 rotates with a period equal to the modulator cycle 85. A typical value of the modulator cycle 85 is 100 ms. So, by using a source setup (means 15), a nozzle 75 and an energy modulator, one can send in the beam direction 5 a charged particle beam 10 that is time modulated in energy between the minimum 70 and maximum 65 energy value. A range modulator is preferably designed for providing a flat SOBP and the minimum 70 and maximum 65 energy values are preferably chosen such that the WED to be measured (ie the reference point 40) falls in the flat SOBP region. Other types of range modulators which do not provide a flat SOBP (for example tilted SOBP) could be used for the invention as well.

A charged particle beam 10 that is time modulated in energy induces in a body 20 a time dependent dose rate 60. When a modulator wheel 80 is used, the induced time dependent dose rate 60 is typically periodic in time with a period equal to the modulator cycle 85. At different positions lying in a body 20 and along a beam direction 5 of a charged particle beam 10 that is time modulated in energy, the shape of the corresponding time dependent dose rates 60 versus time changes. These different shapes are the characteristic patterns 105 referred notably in Hsiao-Ming Lu's publication. As a further illustration, figure 3 shows time dependent dose rates 60 calculated at twenty-one different depths along a beam direction of an energy modulated charged particle beam 10 when these twenty-one different depths correspond to depths in a water tank (the different curves correspond to increasing depths along the vertical arrow of figure 3). As shown in Hsiao-Ming Lu's publication, we observe different characteristic patterns at the different depths.

As one sends a charged particle beam 10 that is time modulated in energy between a minimum energy value 70 and a maximum energy value 65, a time dependent dose rate 60 is induced along a beam direction 5 of this charged particle beam 10. If the minimum 70 and maximum 65 energy values are chosen as explained above, a charged particle beam detector 50 placed at the reference point 40 presents a time dependent response 55 that typically consists of stepwise structures spaced in time by the modulator cycle 85 if a modulator wheel 80 is used to produce the energy modulated charged particle beam 10. Similar to the time dependent dose rate 60, the time dependent responses 55 of a charged particle beam detector 50 present characteristic patterns when said charged particle beam detector 50 is placed at different positions along a beam direction 5 of a charged particle beam 10 that is time modulated in energy. In step e) of the method of the invention, one has to acquire a time dependent response 55 of a charged particle beam detector 50. The way to obtain the time dependent response 55 of a charged particle beam detector 50 is known from the one skilled in the art (see for instance Hsiao-Ming Lu's publication, page 1416).

The inventors have found that it is possible to relate the WED corresponding to the distance 35 between the entrance point 30 and the reference point 40 to a value of a statistical parameter 100 of the time dependent response 55 of the charged particle beam detector 50, when said charged particle beam detector 50 is subjected to a charged particle beam 10 that is time modulated in energy. One can choose different statistical parameters 100 for determining the sought WED. An example is the skewness *sk* whose definition is given below.

From the time dependent response 55 of a charged particle beam detector 50, one can define the following parameters. The sum, S, is given by:

$$S = \sum_{i=i_1}^{i_N} v_i \text{ (Eq. 2)}$$

when the time dependent response 55 is a discrete signal and by

$$S = \int_{t_1}^{t_N} v(t) \, \mathrm{d}t \quad \text{(Eq. 3)}$$

when the time dependent response 55 is a continuous signal. In these equations, $v$ stands for the time dependent response 55 of the charged particle beam detector 50. In equation (Eq. 2), the index $i_1$ (resp. $i_N$) is the index of the first (resp. last) reading of the time dependent response 55. When the time dependent response 55 is continuous, $t_1$ (resp. $t_N$) is the time of the beginning (resp. end) of its acquisition.

The mean time of occurrence, m, of the time dependent response 55 is defined by:

$$m = \frac{1}{S} \sum_{i_1}^{i_N} v_i \, t_i \text{ (Eq. 4),}$$

when the time dependent response 55 is a discrete signal and by

$$m = \frac{1}{S} \int_{t_1}^{t_N} v(t) \, t \, \mathrm{d}t \text{ (Eq. 5)}$$

when the time dependent response 55 is a continuous function, S being given by equations (Eq. 2) (discrete case) or (Eq. 3) (continuous case). The root mean square width 101, $\sigma$, is given by:

$$\sigma = \sqrt{\frac{1}{S}\sum_{i_1}^{i_N} v_i(t_i - m)^2} \quad \text{(Eq. 6)}$$

when the time dependent response 55 is a discrete signal and by

$$\sigma = \sqrt{\frac{1}{S}\int_{t_1}^{t_N} v(t)(t - m)^2} \quad \text{(Eq. 7)}$$

when the time dependent response 55 is a continuous function. The skewness if given by:

$$sk = \frac{1}{S\sigma^3}\sum_{i_1}^{i_N} v_i(t_i - m)^3 \quad \text{(Eq. 8)}$$

in the discrete case, and by

$$sk = \frac{1}{S\sigma^3}\int_{t_1}^{t_N} v(t)(t - m)^3 \quad \text{(Eq. 9).}$$

The parameters $S$ and $m$ entering equations (Eq. 6) to (Eq. 9) are given by equations (Eq. 2) and (Eq. 4) in the discrete case and by (Eq. 3) and (Eq. 5) in the continuous case.

When a modulator wheel 80 is used, the time dependent response 55 of a charged particle beam detector 50 typically presents periodic patterns 105 separated in time by a period equal to the modulator cycle 85 as shown in figure 5 (top graph). Rather than acquiring the whole signal of the time dependent response 55 of a charged particle beam detector 50 before determining the value of a statistical parameter 100 such as the skewness, one skilled in the art can imagine recording its response during only one of its period by the use of synchronizing means. Then, one can apply equation (Eq. 8) or (Eq. 9) to determine the value of the skeness corresponding to said period. To increase statistics and precision, the measurement of the time dependent response can also be performed over multiple cycles of the range modulator.

As the time dependent response 55 of a charged particle beam detector 50 subjected to an energy modulated charged particle beam 10 typically presents a stepwise structure, one can also isolate the different steps from the background measured level before applying equations (Eq. 8) or (Eq. 9) when the time dependent response 55 is acquired during a time longer than the modulator cycle 85. One can also calculate values of a statistical parameter 100 corresponding to different periods of the time dependent response 55, and after average these values over the different periods to obtain an average value of the chosen statistical parameter 100. This procedure is particularly useful when the time dependent response 55 has a low signal to noise level. Alternatively, the data acquisition system can operate in synchrony with the rotation of the range modulator wheel 80 so as to obtain an accumulated time dependent response signal.

Figure 5 shows the time dependent response 55 of a charged particle beam detector 50 that is a PTW Model T60012 semiconductor diode when such a diode is placed at a given depth (16.5 cm is this example) in a water phantom and subjected to a charged particle beam 10 that is time modulated in energy. In this example, the time dependent response 55 of the charged particle beam detector 50 is a time dependent negative voltage. The upper part of figure 5 shows ten patterns corresponding to ten modulator cycles 85 of the modulator wheel 80 that is used for producing the energy modulated charged particle beam. The modulator cycle 85 is equal to 100 ms in this example. The middle graph of figure 5 is a zoom of the fourth cycle whereas the lower graph shows only the beginning of the same fourth cycle. The used diode presents a disk of area 1 mm$^2$ and a thickness equal to 2,5 $\mu$m (2,5 $\mu$m is the thickness of the charged particle beam detector 50 along a beam direction 5 of the charged particle beam 10). The diode is connected to a transimpedance amplifier 120 whose details are given below.

When a signal such as the one depicted in figure 5 is acquired, one preferably first isolates the different patterns 105 or real signals from the background level. As an example, the first pattern 105 can be deemed to begin when a voltage reading is more negative than a given discrimination level. After, one can take advantage of the stable mean time between each pattern 105 that is typically equal to the modulator cycle 85 to remove occasional spurious pulses between each pattern. Knowing the value of the modulator cycle 85, one does not need to seek the second pattern 105 until preferably 98 ms after the first one if the modulator cycle 85 is equal to 100 ms. Continuing thus, one can identify all the ten patterns 105 of figure 5 and determine for each of them the value of a statistical parameter 100 such as the skewness. In this case, the index $i_1$ (resp. $i_N$) entering equations (Eq. 2), (Eq. 4), (Eq. 6), and (Eq. 8) is the index of the first (resp. last) reading of each pattern 105 isolated from the background level. Knowing the different values of a statistical parameter 100 corresponding to the different patterns, one can easily calculate an averaged value of such a statistical parameter 100. Preferably, one can acquire the time dependent response 55 during a time that is longer than ten modular cycles 85 and determine more than ten values of a statistical parameter 100 before calculating an averaged value of such a statistical parameter 100.

If one acquires the time dependent response 55 of a charged particle beam detector 50 that is positioned at different depths in a water tank and that is subjected to a charged particle beam 10 that is time modulated in energy, one can deduce for each depth the value of a statistical parameter 100 such as the skewness by using equations (Eq. 8) or (Eq. 9). By following this procedure, the inventors have found that such a statistical parameter 100 is smoothly and monotonically related to the depth of the charged particle beam detector 50 in the water tank. As a consequence, the dependence of said statistical parameter 100 to the depth of the charged particle beam detector 50 in the water tank can be well represented by a curve and such a curve can be used as a calibration curve 95. Indeed, the depths of the charged particle beam detector 50 with respect to the entrance point of a charged particle beam 10 in the water tank can be identified to WEDs. Step g) of the method of the invention aims at providing such a calibration curve 95. There are different possibilities to obtain such a calibration curve 95. One can measure the time dependent response 55 of a charged particle beam detector 50 that is positioned at different depths in a water tank and that is subjected to a charged particle beam 10 that is time modulated in energy. Or, the calibration curve 95 can be obtained from calculations that predict the time dependent response 55 of a charged particle beam detector 50 placed at different depths in a water tank and exposed to an energy modulated charged particle beam 10. The conditions of the calibration procedure need to be the same as those used during the acquisition of the time dependent response 55 of the charged particle beam detector 50 placed at the reference point 40. Instead of a calibration curve 95, one could establish a calibration table.

Finally (step h) of the method of the invention), by using the calibration curve 95 provided in step g), one can determine the WED corresponding to the value of the statistical parameter 100 determined in step f). As an illustration and referring to figure 6, if the value of the statistical parameter 100 determined in step f) is equal to a value that we name 160, the corresponding WED is equal to a value that we name 165.

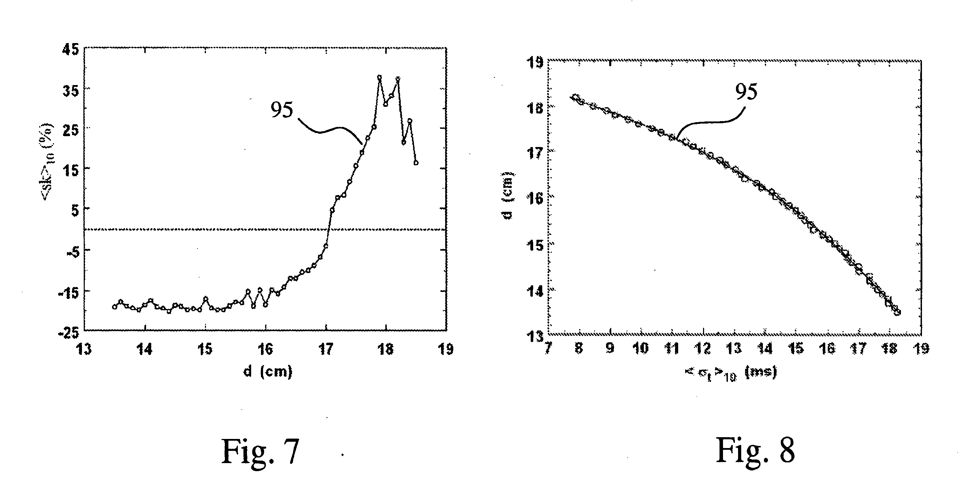

Preferably, the statistical parameter 100 that is used for determining the WED is the root mean square width 101 of the time dependent response 55 of the charged particle beam detector 50. The root mean square width 101 can be obtained from equations (Eq. 2), (Eq. 4), (Eq. 6) (discrete case), or (Eq. 3), (Eq. 5), (Eq. 7) (continuous case). When using such a statistical parameter 100, the region of good sensitivity is large. This is illustrated in figures 7 and 8 that show two measured calibration curves 95 corresponding to the variations of two different statistical parameters 100 with respect to the depth of a charged particle beam detector 50 in a water tank. Figure 7 (respectively 8) shows a calibration curve 95 when the statistical parameter 100 is a skewness (respectively root mean square width 101). From these two figures, we see that the region of good sensitivity is narrower for the skewness as the slope of the curve of figure 7 is smaller than the slope of the curve of figure 8.

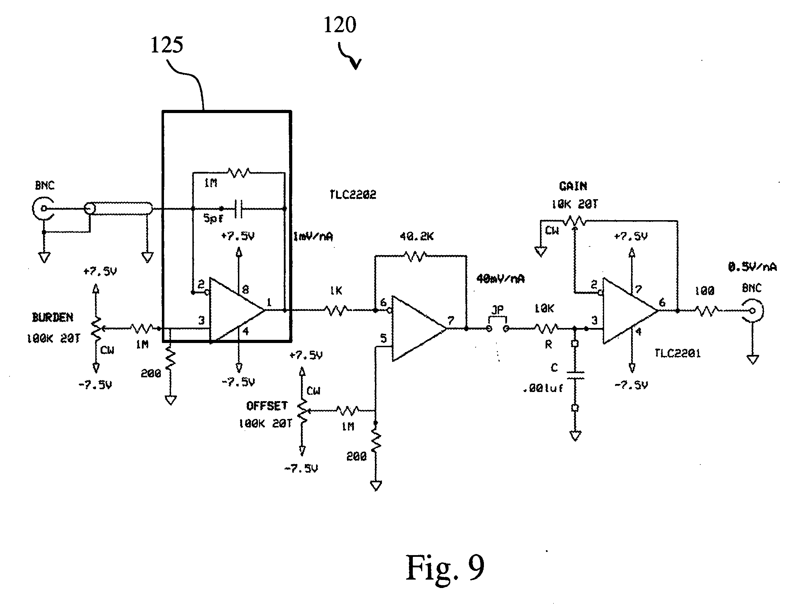

Different charged particle beam detectors 50 can be used to carry out the method of the invention. In a preferred embodiment, the charged particle beam detector 50 comprises a semiconductor diode 110. Ordinary semiconductor diodes 110 make good charged particle beam detectors 50 and present some advantages: small, rugged, and inexpensive. In yet another embodiment, the charged particle beam detector 50 comprises a PTW Model T60012 diode connected to a transimpedance amplifier 120. In yet another preferred embodiment, said transimpedance amplifier 120 has three stages. The first stage is a first transimpedance amplifier 125 with an output of 1 mV/nA and a response time of 5 $\mu$s dominated by the feedback circuit. The next stage is an inverting voltage amplifier with a gain of 40. The output stage gain was set for a total gain of 0.5 V/nA using a precise current source. Since it is very convenient for the quiescent DC offset to be zero and stable, regulated on-board tracking 7.5 V regulators are provided. The amplifier has two DC adjustments. 'OFFSET' is set to null the output when the input is open. 'BURDEN' is set to null the output when the input is grounded through preferably 10 k$\Omega$. Such a transimpedance amplifier, which is illustrated in figure 9, can be connected to an oscilloscope through a coaxial cable.

The calibration curve 95 that is used to link the value of the statistical parameter 100 to the WED (step h of the method of the invention) of the method of the invention) can be obtained experimentally. Preferably, such an experimental determination comprises the following steps:

i. positioning said charged particle beam detector 50 at a given position 170 in a water or water-equivalent calibration phantom 115;

ii. sending to said charged particle beam detector 50 the charged particle beam 10 that is time modulated in energy between said minimum 70 and said maximum 65 energy value;

iii. acquiring a time dependent response 55 of said charged particle beam detector 50;

iv. determining a value of said statistical parameter 100 from said time dependent response 55;

v. determining a water equivalent depth 1, said water equivalent depth 1 being the distance between the entrance point of said charged particle beam 10 in said calibration phantom 115 and said given position 170;

vi. repeating steps i. to v. n times, n being an integer larger than one;

vii. establishing a calibration curve 95 that relates the values of the statistical parameter 100 determined in step iv. and the water equivalent depth 1 determined in step v..

When the statistical parameter 100 is a root mean square width 101, the calibration curve 95 of step vii is preferably a cubic polynomial curve. Adding higher order terms does not improve the fit quality and the precision significantly.

In another embodiment, the charged particle beam detector 50 comprises an array of semiconductor diodes 110 placed transversally with respect to the charged particle beam 10. Typically, the semiconductor diodes 110 are then spread over a few cm$^2$, preferably on a flex circuit attached to a rectal balloon. By using such an array, one can deduce for each semiconductor diode 110 the value of a statistical parameter 100, preferably a root mean square width 101 by using the method of the invention described above. By comparing the different values corresponding to the different semiconductor diodes 110, one can evaluate the spatial variation of the WED in the body 20 or obtain a mean value over the area covered by the array of semiconductor diodes 110.

According to a second aspect of the invention, the inventors propose to use a device for determining the WED between an entrance point 30 and a reference point 40. Such a device is shown in figure 10 with other elements. The device of the invention comprises a unit such as a computer 200 with a set of subunits or software modules that implement various steps of the method of the invention. The computer 200 can be an ordinary, single processor personal computer. The different software modules described below can be included in different computers or different units rather than in a single computer 200. The computer 200 also includes an internal memory (not shown in figure 10) for storing computer program instructions which control how a processing unit within the computer 200 accepts, transforms, and outputs data. The internal memory includes both a volatile and a non-volatile portion. Those skilled in the art will recognize that the internal memory can be supplemented with computer memory media, such as compact disk, flash memory cards, magnetic disc drives.

Before the computer 200 can determine a WED 1 of a charged particle beam 10 that is sent to a body 20, a minimum 70 and a maximum 65 energy value are provided to means 15 (or source setup) by a treatment planning 280 as an example. More precisely, these energy values (70, 65) are sent to a control system 320 of the source setup. The source setup (or means 15) is able to send a charged particle beam 10 that is time modulated in energy between these minimum 70 and maximum 65 energy values along a beam trajectory 5. The minimum 70 and maximum 65 energy values have been defined above. A charged particle beam 10 with the maximum (respectively minimum) energy value 65 (respectively 70) penetrates the body 20 along a beam direction 5 till a depth 45 that is equal or larger (respectively smaller) than the reference distance 35. The depth 45 till which a charged particle beam 10 of a given energy penetrates has been defined above with figure 2.

The device of the invention comprises a charged particle beam detector 50 that is placed at the reference point 40. When the charged particle beam detector 50 is subjected to a charged particle beam 10 that is time modulated in energy between the minimum 70 and the maximum 65 energy values, it presents a time dependent response 55. This time dependent response 55 can be measured by specific electronic components known by those skilled in the art. If one uses an ionization chamber for the charged particle beam detector 50, one can use for instance a scanning system that samples, in time, the ionization current generated in an ionization chamber. Means 230 of the device of the invention allows one to acquire the time dependent response 55 of the charged particle beam detector 50.

From this time dependent response 55, means 240 (a subunit of the computer 200) of the device of the invention determines a value of a statistical parameter 100, for instance the skewness that is given by equations (Eq. 8) for a discrete signal and by equation (Eq. 9) when the signal is continuous. Preferably the statistical parameter 100 is a root mean square width 101 given by equations (Eq. 6) or (Eq. 7). The device 200 comprises preferably a library of calibration curves 95 obtained for various beam modulations, i.e. for various values of minimum 75 and maximum 65 energies. As discussed above, each of these calibration curves 95 expresses a relationship between values of a statistical parameter 100 and WEDs. A subunit 250 selects a calibration curve 95 from the library that is in correspondence with the minimum and maximum energy values. Means 260 (another subunit of the computer 200) can eventually determine from this selected calibration curve 95 the WED corresponding to the statistical parameter 100 of the time dependent response 55. This result can optionally be sent to a screen 270.

In a preferred embodiment, the charged particle beam detector 50 comprises a semiconductor diode 110. In another embodiment, such a semiconductor diode 110 is connected to the input of a transimpedance amplifier 120 instead of using a usual current integrator. The time dependent response 55 is then measured at the ouput of the transimpedance amplifier 120. In another preferred embodiment, such a transimpedance amplifier 120 has three stages (see figure 9): a first transimpedance amplifier 125, an inverting voltage amplifier with a gain ranging between 35 and 45, and an output stage whose gain is set to obtain a total gain ranging between 0.4 V / nA and 0.6 V / nA. In yet another preferred embodiment, the first stage 125 of such a transimpedance amplifier 120 has an output of 1 mV/nA and a response time of 5 μs; the second stage (inverting voltage amplifier) has a gain of 40, and the output stage is set for a total gain of 0.5 V/nA. The charged particle beam detector 50 preferably comprises an array of semiconductor diodes 110 placed transversally with respect to the charged particle beam 10.

According to a last aspect of the invention, a program is proposed. This program allows one to determine a water equivalent depth between an entrance point 30 and a reference point 40, said entrance point 30 lying on an external surface of a body 20, said reference point 40 being a point at which a charged particle beam detector 50 is placed, said program comprising :

- a software module 430 for acquiring a time dependent response 55 of said charged particle beam detector 50;
- a software module 440 for determining a value of a statistical parameter 100 of said time dependent response 55;
- a software module 450 for loading a pre-determined calibration curve 95 expressing a relationship between values of said statistical parameter 100 and water equivalent depths 1;
- a software module 460 for determining from said calibration curve 95 the water equivalent depth 1 corresponding to the value of the statistical parameter 100 of the time dependent response 55 of the charged particle beam detector 50 placed at the reference point 40.

We now present some results obtained with the method of the invention. As a reminder, two examples of calibration curve 95 are shown in figure 7 and 8 that are now detailed. A proton beam 10 that is modulated in energy has been sent to a water tank. The time dependent response 55 of a PTW Model T60012 diode that is subjected to such a charged particle beam 10 and that is positioned at different depths in a water tank has been recorded during ten modulator cycles 85. At each depth and for each modulator cycles 85, the value of a statistical parameter 100 is determined. In figure 7, the statistical parameter is the skewness given by equation (Eq. 8) whereas figure 8 corresponds to the case where the statistical parameter is a root mean square width 101 given by equation (Eq. 6). Then, an averaged value of these two statistical parameters 100 (denoted by $\langle sk \rangle_{10}$ in figure 7 and by $\langle \sigma_t \rangle_{10}$ in figure 8) can be calculated for each depth by taking the average of the values of the corresponding statistical parameters 100 determined for each modulator cycle 85. The circles in figure 7 and 8 show the averaged values of these statistical parameters 100 at different depths. In figure 8, the continuous curve is a fit corresponding to a cubic polynomial. This figure shows the smooth and monotonic dependence of the root mean square width 100 with depth (or WED).

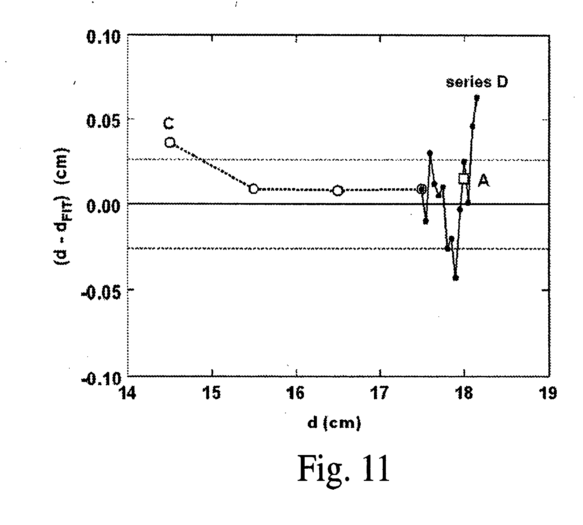

To obtain the experimental calibration curves 95 of figure 7 and 8, the inventors have used a PTW Model T60012 diode connected to a transimpedance amplifier 120 as the one shown in figure 9. Using the same charged particle beam detector 50 connected to the same transimpedance amplifier 120, the inventors have carried out in a water tank three series of measurements that we name A, C and D. At the beginning of each series, the charged particle beam detector 50 was driven to 19 cm and subsequently moved upstream to avoid backlash, if any. For each series, the charged particle beam detector 50 was subjected at different known depths to an energy modulated proton beam 10, and its time dependence response 55 was measured and recorded during ten modulator cycles. For each depth and each modulator cycle, the value of a root mean square width 101 was determined by using equations (Eq. 2), (Eq. 4), and (Eq. 6). Then, for each depth, an averaged value of root mean square width 101 was determined by taking the average of the individual values of root mean square widths 101 corresponding to the ten modulator cycles. By using the calibration curve of figure 8, the inventors have determined the corresponding WEDs or depths in the water tank. Series A corresponds to a preliminary exploration (one point of measurement); series C corresponds to different positions of the charged particle beam detector 50 separated by 1 cm steps; series D relates to measurements in 0.5 mm steps. Figure 11 compares the differences between the actual depth of the charged particle beam detector 50 and the depth estimated from the measured time dependence responses 55 of the charged particle beam detector 50 by using the method of the invention (open squares : series A, open circles : series C, and filled circles : series D). The root mean square scatter of these nineteen points about zero is 0.026 cm (dashed lines in figure 11); such a value represents the precision of the method of the invention that is better than the 1 mm of the Lu's method. With more than ten patterns for determining the value of the root mean square width 101 corresponding to each depth in the water tank, one could expect to increase the precision of the method of the invention.

By using the method of the invention, one can verify the particle range in-vivo, and possibly modify the parameters of a treatment planning after such a determination of the WED.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features. Reference numerals in the claims do not limit their protective scope. Use of the verbs "to comprise", "to include", "to be composed of", or any other variant, as well as their respective conjugations, does not exclude the presence of elements other than those stated. Use of the article "a", "an" or "the" preceding an element does not exclude the presence of a plurality of such elements.

**Claims**

1. A method for determining a water equivalent depth (1) between an entrance point (30) and a reference point (40), said entrance point (30) lying on an external surface of a body (20), said reference point (40) being a point at which a charged particle beam detector (50) is placed,
   said method Comprising the steps of :

   a) providing means for delivering a charged particle beam (10) to said body (20) in a beam direction (5) defined from said entrance point (30) to said reference point (40);
   b) choosing a maximum energy value (65) such that a charged particle beam (10) having said maximum energy value (65) and being sent in said beam direction (5) penetrates the body (20) from said entrance point (30) up to at least said reference point (40);
   c) choosing a minimum energy value (70) such that a charged particle beam (10) having said minimum energy value (70) and being sent in said beam direction (5) penetrates the body (20) from said entrance point (30) and is stopped in said body (20) before reaching said reference point (40);
   d) sending in said beam direction (5) a charged particle beam (10) that is time modulated in energy between said minimum energy value (70) and said maximum energy value (65);
   e) acquiring a time dependent response (55) of the charged particle beam detector (50);
   **characterized in that** said method further comprises the steps of :

   f) determining a value of a statistical parameter (100) of said time dependent response (55);
   g) providing a pre-determined calibration curve (95), expressing a relationship between values of said statistical parameter (100) and water equivalent depths (1);
   h) determining from said calibration curve (95) the water equivalent depth (1) between the entrance point (30) and the reference point (40) corresponding to the value of the statistical parameter (100) determined in step f);
   whereby the delivery of the charged particle beam has no therapeutic effect.

2. A method according to claim 1 **characterized in that** said statistical parameter (100) is a root mean square width (101) of said time dependent response (55).

3. A method according to claim 1 or 2 **characterized in that** said reference point (40) is positioned inside said body (20).

4. A method according to claim 1 or 2 **characterized in that** said reference point (40) is positioned outside said body (20).

5. A method according to any of the previous claims **characterized in that** the charged particle beam detector (50) comprises a semiconductor diode (110) for detecting the charged particle beam (10).

6. A method according to claim 5 **characterized in that** said semiconductor diode (110) is connected to an input of a transimpedance amplifier (120), and **in that** said time dependent response (55) is measured at an output of said transimpedance amplifier (120).

7. A method according to claim 6 **characterized in that** said transimpedance amplifier (120) has three stages:

   - a first transimpendance amplifier (125),
   - an inverting voltage amplifier with a gain ranging between 35 and 45,
   - an output stage whose gain is set to obtain a gain between the input and the output of said transimpedance amplifier (120) ranging between 0.4 V / nA and 0.6 V / nA.

8. A method according to any of the previous claims **characterized in that** the pre-determined calibration curve (95) is obtained from the following steps:

i. positioning said charged particle beam detector (50) at a given position (170) in a water or water-equivalent calibration phantom (115);

ii. sending to said charged particle beam detector (50) the charged particle beam (10) that is time modulated in energy between said minimum (70) and said maximum (65) energy value;

iii. acquiring a time dependent response (55) of said charged particle beam detector (50);

iv. determining a value of said statistical parameter (100) from said time dependent response (55);

v. determining a water equivalent depth (1), said water equivalent depth (1) being the distance between the entrance point of said charged particle beam (10) in said calibration phantom (115) and said given position (170);

vi. repeating steps i. to v. n times, n being an integer larger than one;

vii. establishing a calibration curve (95) that relates the values of the statistical parameter (100) determined in step iv. and the water equivalent depth (1) determined in step v..

9. A method according to any of the previous claims **characterized in that** the charged particle beam detector (50) comprises an array of semiconductor diodes (110) placed transversally with respect to the charged particle beam (10).

10. A device for determining a water equivalent depth (1) between an entrance point (30) and a reference point (40), said entrance point (30) lying on an external surface of a body (20), said device comprising :

- a charged particle beam detector (50) that is placed at the reference point (40);
- means (230) adapted to acquire a time dependent response (55) of said charged particle beam detector (50), induced by an energy modulated charged particle beam (10),

**characterized in that** said device further comprises :

- means (240) adapted to determine a value of a statistical parameter (100) of said time dependent response (55);
- means (250) adapted to load a pre-determined calibration curve (95) expressing a relationship between values of said statistical parameter (100) and water equivalent depths (1);
- means (260) adapted to determine from said calibration curve (95) the water equivalent depth (1) corresponding to the value of the statistical parameter (100) of the time dependent response (55) of the charged particle beam detector (50) placed at the reference point (40).

11. A device according to claim 10 **characterized in that** the charged particle beam detector (50) comprises a semiconductor diode (110).

12. A device according to claim 11 **characterized in that** said semiconductor diode (110) is connected to an input of a transimpedance amplifier (120), and **in that** said time dependent response (55) is measured at an output of said transimpedance amplifier (120).

13. A device according to claim 12 **characterized in that** the charged particle beam detector (50) comprises an array of semiconductor diodes (110) placed transversally with respect to the charged particle beam (10).

14. A computer program for determining a water equivalent depth (1) between an entrance point (30) and a reference point (40), said entrance point (30) lying on an external surface of a body (20), said reference point (40) being a point at which a charged particle beam detector (50) is placed, said program comprising :

- a software module (430) configured to acquire a time dependent response (55) of said charged particle beam detector (50);

**characterized in that** said program further comprises :

- a software module (440) configured to determine a value of a statistical parameter (100) of said time dependent response (55);
- a software module (450) configured to load a pre-determined calibration curve (95) expressing a relationship between values of said statistical parameter (100) and water equivalent depths (1);
- a software module (460) configured to determine from said calibration curve (95) the water equivalent depth (1) corresponding to the value of the statistical parameter (100) of the time dependent response (55) of the charged particle beam detector (50) placed at the reference point (40).

**Patentansprüche**

1. Verfahren zum Bestimmen einer Wasseräquivalent-Tiefe (1) zwischen einem Eintrittspunkt (30) und einem Referenzpunkt (40), wobei der Eintrittspunkt (30) auf einer äußeren Oberfläche eines Körpers (20) liegt und der Referenzpunkt (40) ein Punkt ist, an dem ein Detektor (50) für einen Strahl geladener Teilchen platziert ist, wobei das Verfahren die folgenden Schritte umfasst:

a) Bereitstellen von Mitteln zum Abgeben eines Strahls geladener Teilchen (10) an den Körper (20) in einer Strahlrichtung (5), die von dem Eintrittspunkt (30) bis zu dem Referenzpunkt (40) definiert ist;
b) Wählen eines Maximalenergiewerts (65), so dass ein Strahl geladener Teilchen (10), der den Maximalenergiewert (65) besitzt und in die Strahlrichtung (5) gesendet wird, in den Körper (20) von dem Eintrittspunkt (30) mindestens bis zu dem Referenzpunkt (40) eindringt;
c) Wählen eines Minimalenergiewerts (70), so dass ein Strahl geladener Teilchen (10), der den Minimalenergiewert (70) besitzt und in die Strahlrichtung (5) gesendet wird, in den Körper (20) von dem Eintrittspunkt (30) aus eindringt und in dem Körper (20) gestoppt wird, bevor er den Referenzpunkt (40) erreicht;
d) Senden eines Strahls geladener Teilchen (10), der in der Energie zwischen dem Minimalenergiewert (70) und dem Maximalenergiewert (65) zeitlich moduliert wird, in die Strahlrichtung (5);
e) Erlangen einer zeitabhängigen Antwort (55) des Detektors (50) für den Strahl geladener Teilchen;
**dadurch gekennzeichnet, dass** das Verfahren ferner die folgenden Schritte umfasst:

f) Bestimmen eines Werts eines statistischen Parameters (100) der zeitabhängigen Antwort (55);
g) Bereitstellen einer vorbestimmten Kalibrierungskurve (95), die eine Beziehung zwischen Werten des statistischen Parameters (100) und Wasseräquivalent-Tiefen (1) ausdrückt;
h) Bestimmen der Wasseräquivalent-Tiefe (1) zwischen dem Eintrittspunkt (30) und dem Referenzpunkt (40) aus der Kalibrierungskurve (95) entsprechend dem Wert des statistischen Parameters (100), der in Schritt f) bestimmt wird;

wobei die Abgabe des Strahls geladener Teilchen keinen therapeutischen Effekt hat.

2. Verfahren nach Anspruch 1, **dadurch gekennzeichnet, dass** der statistische Parameter (100) die Wurzel einer mittleren quadratischen Breite (101) der zeitabhängigen Antwort (55) ist.

3. Verfahren nach Anspruch 1 oder 2, **dadurch gekennzeichnet, dass** der Referenzpunkt (40) innerhalb des Körpers (20) positioniert ist.

4. Verfahren nach Anspruch 1 oder 2, **dadurch gekennzeichnet, dass** der Referenzpunkt (40) außerhalb des Körpers (20) positioniert ist.

5. Verfahren nach einem der vorhergehenden Ansprüche, **dadurch gekennzeichnet, dass** der Detektor (50) für den Strahl geladener Teilchen eine Halbleiterdiode (110) umfasst, um den Strahl geladener Teilchen (10) zu detektieren.

6. Verfahren nach Anspruch 5, **dadurch gekennzeichnet, dass** die Halbleiterdiode (110) mit einem Eingang eines Transimpedanzverstärkers (120) verbunden ist und dass die zeitabhängige Antwort (55) an einem Ausgang des Transimpedanzverstärkers (120) gemessen wird.

7. Verfahren nach Anspruch 6, **dadurch gekennzeichnet, dass** der Transimpedanzverstärker (120) drei Stufen hat:

- einen ersten Transimpedanzverstärker (125)
- einen invertierenden Spannungsverstärker mit einer Verstärkung, die sich zwischen 35 und 45 bewegt,
- eine Ausgangsstufe, deren Verstärkung so festgelegt ist, dass sich eine Verstärkung zwischen dem Eingang und dem Ausgang des Transimpedanzverstärkers (120) ergibt, die in einem Bereich von 0,4 V/nA bis 0,6 V/nA liegt.

8. Verfahren nach einem der vorhergehenden Ansprüche **dadurch gekennzeichnet, dass** die vorbestimmte Kalibrierungskurve (95) aus den folgenden Schritten erhalten wird:

i. Positionieren des Detektors (50) für den Strahl geladener Teilchen an einer gegebenen Position (170) in einem Wasser- oder Wasseräquivalent-Kalibrierphantom (115);

ii. Senden des Strahls geladener Teilchen (10), der in der Energie zwischen dem Minimalenergiewert (70) und dem Maximalenergiewert (65) zeitlich moduliert wird, an den Detektor (50) für den Strahl geladener Teilchen;

iii. Erlangen einer zeitabhängigen Antwort (55) des Detektors (50) für den Strahl geladener Teilchen;

iv. Bestimmen eines Werts eines statistischen Parameters (100) aus der zeitabhängigen Antwort (55);

v. Bestimmen der Wasseräquivalent-Tiefe (1), wobei die Wasseräquivalent-Tiefe (1) der Abstand zwischen dem Eintrittspunkt des Strahls geladener Teilchen (10) in dem Kalibrierphantom (115) und der gegebenen Position (170) ist;

vi. n-faches Wiederholen der Schritte i. bis v., wobei n eine ganze Zahl größer als eins ist;

vii. Erstellen einer Kalibrierungskurve (95), die die Werte des statistischen Parameters (100), die in Schritt iv. bestimmt werden, und die Wasseräquivalent-Tiefe (1), die in Schritt v. bestimmt wird, in Beziehung bringt.

9. Verfahren nach einem der vorhergehenden Ansprüche, **dadurch gekennzeichnet, dass** der Detektor (50) für den Strahl geladener Teilchen eine Anordnung von Halbleiterdioden (110) umfasst, die quer in Bezug zu dem Strahl geladener Teilchen (10) platziert ist.

10. Vorrichtung zum Bestimmen einer Wasseräquivalent-Tiefe (1) zwischen einem Eintrittspunkt (30) und einem Referenzpunkt (40), wobei der Eintrittspunkt (30) auf einer äußeren Oberfläche eines Körpers (20) liegt und die Vorrichtung Folgendes umfasst:

- einen Detektor (50) für einen Strahl geladener Teilchen, der an dem Referenzpunkt (40) platziert ist;
- Mittel (230), die dazu ausgelegt sind, eine zeitabhängige Antwort (55) des Detektors (50) für den Strahl geladener Teilchen, die durch einen energiemodulierten Strahl geladener Teilchen (10) hervorgerufen wird, zu erlangen;

**dadurch gekennzeichnet, dass** die Vorrichtung ferner Folgendes umfasst:

- Mittel (240), die dazu ausgelegt sind, einen Wert eines statistischen Parameters (100) der zeitabhängigen Antwort (55) zu bestimmen;
- Mittel (250), die dazu ausgelegt sind, eine vorbestimmte Kalibrierungskurve (95), die eine Beziehung zwischen Werten des statistischen Parameters (100) und Wasseräquivalent-Tiefen (1) ausdrückt, zu laden;
- Mittel (260), die dazu ausgelegt sind, die Wasseräquivalent-Tiefe (1) aus der Kalibrierungskurve (95) entsprechend dem Wert des statistischen Parameters (100) der zeitabhängigen Antwort (55) des Detektors (50) für den Strahl geladener Teilchen, der an dem Referenzpunkt (40) platziert ist, zu bestimmen.

11. Vorrichtung nach Anspruch 10, **dadurch gekennzeichnet, dass** der Detektor (50) für den Strahl geladener Teilchen eine Halbleiterdiode (110) umfasst.

12. Vorrichtung nach Anspruch 11, **dadurch gekennzeichnet, dass** die Halbleiterdiode (110) mit einem Eingang eines Transimpedanzverstärkers (120) verbunden ist und dass die zeitabhängige Antwort (55) an einem Ausgang des Transimpedanzverstärkers (120) gemessen wird.

13. Vorrichtung nach Anspruch 12, **dadurch gekennzeichnet, dass** der Detektor (50) für den Strahl geladener Teilchen eine Anordnung von Halbleiterdioden (110) umfasst, die quer in Bezug zu dem Strahl geladener Teilchen (10) platziert ist.

14. Computerprogramm zum Bestimmen einer Wasseräquivalent-Tiefe (1) zwischen einem Eintrittspunkt (30) und einem Referenzpunkt (40), wobei der Eintrittspunkt (30) auf einer äußeren Oberfläche eines Körpers (20) liegt und der Referenzpunkt (40) ein Punkt ist, an dem ein Detektor (50) für einen Strahl geladener Teilchen platziert ist, wobei das Programm Folgendes umfasst:

- ein Softwaremodul (430), das dazu konfiguriert ist, eine zeitabhängige Antwort (55) des Detektors (50) für den Strahl geladener Teilchen zu erlangen;

**dadurch gekennzeichnet, dass** das Programm ferner Folgendes umfasst:

- ein Softwaremodul (440), das dazu konfiguriert ist, einen Wert eines statistischen Parameters (100) der zeitabhängigen Antwort (55) zu bestimmen;
- ein Softwaremodul (450), das dazu konfiguriert ist, eine vorbestimmte Kalibrierungskurve (95), die einen Zusammenhang zwischen Werten des statistischen Parameters (100) und Wasseräquivalent-Tiefen (1) ausdrückt, zu laden;

- ein Softwaremodul (460), das dazu konfiguriert ist, die Wasseräquivalent-Tiefe (1) aus der Kalibrierungskurve (95) entsprechend dem Wert des statistischen Parameters (100) der zeitabhängigen Antwort (55) des Detektors (50) für den Strahl geladener Teilchen, der an dem Referenzpunkt (40) platziert ist, zu bestimmen.

## Revendications

1. Procédé pour déterminer une profondeur d'équivalent en eau (1) entre un point d'entrée (30) et un point de référence (40), ledit point d'entrée (30) se situant sur une surface externe d'un corps (20), ledit point de référence (40) étant un point au niveau duquel est placé un détecteur de faisceau de particules chargées (50), ledit procédé comprenant les étapes consistant :

   a) à fournir des moyens pour délivrer un faisceau de particules chargées (10) audit corps (20) suivant une direction de faisceau (5) définie à partir dudit point d'entrée (30) jusqu'audit point de référence (40) ;
   b) à choisir une valeur d'énergie maximum (65), de manière telle qu'un faisceau de particules chargées (10) ayant ladite valeur d'énergie maximum (65) et étant envoyé dans ladite direction de faisceau (5) pénètre le corps (20) à partir dudit point d'entrée (30) jusqu'au moins audit point de référence (40) ;
   c) à choisir une valeur d'énergie minimum (70), de manière telle qu'un faisceau de particules chargées (10) ayant ladite valeur d'énergie minimum (70) et étant envoyé dans ladite direction de faisceau (5) pénètre le corps (20) à partir dudit point d'entrée (30) et soit stoppé dans ledit corps (20) avant d'atteindre ledit point de référence (40) ;
   d) à envoyer dans ladite direction de faisceau (5), un faisceau de particules chargées (10) qui est modulé en énergie, dans le temps, entre ladite valeur d'énergie minimum (70) et ladite valeur d'énergie maximum (65) ;
   e) à acquérir une réponse (55) - en fonction du temps - du détecteur (50) du faisceau de particules chargées ;
   **caractérisé en ce que** ledit procédé comprend en outre les étapes consistant :

   f) à déterminer une valeur d'un paramètre statistique (100) de ladite réponse (55) en fonction du temps ;
   g) à fournir une courbe d'étalonnage prédéterminée (95) exprimant une relation entre des valeurs dudit paramètre statistique (100) et des profondeurs d'équivalents en eau (1) ;
   h) à déterminer, à partir de ladite courbe d'étalonnage (95), la profondeur d'équivalent en eau (1) entre le point d'entrée (30) et le point de référence (40), ladite profondeur correspondant à la valeur du paramètre statistique (100) déterminé au cours de l'étape f) ;

   où le fait de délivrer le faisceau de particules chargées n'a aucun effet thérapeutique.

2. Procédé selon la revendication 1, **caractérisé en ce que** ledit paramètre statistique (100) est une largeur quadratique moyenne (101) de ladite réponse (55) en fonction du temps.

3. Procédé selon la revendication 1 ou 2, **caractérisé en ce que** ledit point de référence (40) est positionné à l'intérieur dudit corps (20).

4. Procédé selon la revendication 1 ou 2, **caractérisé en ce que** ledit point de référence (40) est positionné à l'extérieur dudit corps (20).

5. Procédé selon l'une quelconque des revendications précédentes, **caractérisé en ce que** le détecteur (50) du faisceau de particules chargées comprend une diode à semiconducteurs (110) pour détecter le faisceau de particules chargées (10).

6. Procédé selon la revendication 5, **caractérisé en ce que** ladite diode à semiconducteurs (110) est connectée à une entrée d'un amplificateur d'adaptation d'impédance (120), et **en ce que** ladite réponse (55) en fonction du temps est mesurée au niveau d'une sortie dudit amplificateur d'adaptation d'impédance (120).

7. Procédé selon la revendication 6, **caractérisé en ce que** ledit amplificateur d'adaptation d'impédance (120) a trois étages :

   - un premier amplificateur d'adaptation d'impédance (125),
   - un amplificateur inverseur de tension ayant un gain variant entre 35 et 45,

- un étage de sortie dont le gain est réglé pour obtenir un gain entre l'entrée et la sortie dudit amplificateur d'adaptation d'impédance (120), ledit gain variant entre 0,4 V / nA et 0,6 V / nA.

8. Procédé selon l'une quelconque des revendications précédentes, **caractérisé en ce que** la courbe d'étalonnage prédéterminée (95) est obtenue à partir des étapes qui suivent, consistant :

   i. à positionner ledit détecteur (50) du faisceau de particules chargées au niveau d'une position donnée (170) dans un fantôme d'étalonnage d'eau ou d'équivalent en eau (115) ;
   ii. à envoyer audit détecteur (50) du faisceau de particules chargées, le faisceau de particules chargées (10) qui est modulé en énergie, dans le temps, entre ladite valeur d'énergie minimum (70) et ladite valeur d'énergie maximum (65) ;
   iii. à acquérir une réponse (55) - en fonction du temps - dudit détecteur (50) du faisceau de particules chargées ;
   iv. à déterminer une valeur dudit paramètre statistique (100) à partir de ladite réponse (55) en fonction du temps ;
   v. à déterminer une profondeur d'équivalent en eau (1), ladite profondeur d'équivalent en eau (1) étant la distance comprise entre le point d'entrée dudit faisceau de particules chargées (10) dans ledit fantôme d'étalonnage (115), et ladite position donnée (170) ;
   vi. à répéter les étapes i. à v. un nombre de fois n, n étant un nombre entier supérieur à un ;
   vii. à établir une courbe d'étalonnage (95) qui concerne les valeurs du paramètre statistique (100) déterminées au cours de l'étape iv. et la profondeur d'équivalent en eau (1) déterminée au cours de l'étape v..

9. Procédé selon l'une quelconque des revendications précédentes, **caractérisé en ce que** le détecteur (50) du faisceau de particules chargées comprend un ensemble de diodes à semiconducteurs (110) placées de façon transversale par rapport au faisceau de particules chargées (10).

10. Dispositif pour déterminer une profondeur d'équivalent en eau (1) entre un point d'entrée (30) et un point de référence (40), ledit point d'entrée (30) se situant sur une surface externe d'un corps (20), ledit dispositif comprenant :

    - un détecteur (50) du faisceau de particules chargées, détecteur qui est placé au niveau du point de référence (40) ;
    - des moyens (230) adaptés pour acquérir une réponse (55) - en fonction du temps - dudit détecteur (50) du faisceau de particules chargées, ladite réponse étant induite par un faisceau de particules chargées (10) modulé en énergie, **caractérisé en ce que** ledit dispositif comprend en outre :
    - des moyens (240) adaptés pour déterminer une valeur d'un paramètre statistique (100) de ladite réponse (55) en fonction du temps ;
    - des moyens (250) adaptés pour charger une courbe d'étalonnage prédéterminée (95) exprimant une relation entre des valeurs dudit paramètre statistique (100) et des profondeurs d'équivalents en eau (1) ;
    - des moyens (260) adaptés pour déterminer, à partir de ladite courbe d'étalonnage (95), la profondeur d'équivalent en eau (1) correspondant à la valeur du paramètre statistique (100) de la réponse (55) - en fonction du temps - du détecteur (50) du faisceau de particules chargées, ledit détecteur étant placé au niveau du point de référence (40).

11. Dispositif selon la revendication 10, **caractérisé en ce que** le détecteur (50) du faisceau de particules chargées comprend une diode à semiconducteurs (110).

12. Dispositif selon la revendication 11, **caractérisé en ce que** ladite diode à semiconducteurs (110) est connectée à une entrée d'un amplificateur d'adaptation d'impédance (120), et **en ce que** ladite réponse (55) en fonction du temps est mesurée au niveau d'une sortie dudit amplificateur d'adaptation d'impédance (120).

13. Dispositif selon la revendication 12, **caractérisé en ce que** le détecteur (50) du faisceau de particules chargées comprend un ensemble de diodes à semiconducteurs (110) placées de façon transversale par rapport au faisceau de particules chargées (10).

14. Programme informatique pour déterminer une profondeur d'équivalent en eau (1) entre un point d'entrée (30) et un point de référence (40), ledit point d'entrée (30) se situant sur une surface externe d'un corps (20), ledit point de référence (40) étant un point au niveau duquel est placé un détecteur (50) du faisceau de particules chargées, ledit programme comprenant :

    - un module logiciel (430) configuré pour acquérir une réponse (55) - en fonction du temps - dudit détecteur (50) du faisceau de particules chargées ;
**caractérisé en ce que** ledit programme comprend en outre :

- un module logiciel (440) configuré pour déterminer une valeur d'un paramètre statistique (100) de ladite réponse (55) en fonction du temps ;
- un module logiciel (450) configuré pour charger une courbe d'étalonnage prédéterminée (95) exprimant une relation entre des valeurs dudit paramètre statistique (100) et des profondeurs d'équivalents en eau (1) ;
- un module logiciel (460) configuré pour déterminer, à partir de ladite courbe d'étalonnage (95), la profondeur d'équivalent en eau (1) correspondant à la valeur du paramètre statistique (100) de la réponse (55) - en fonction du temps - du détecteur (50) du faisceau de particules chargées, ledit détecteur étant placé au niveau du point de référence (40).

Fig. 1

EP 2 497 535 B1

Fig. 4

Fig. 2

Fig. 3

Fig. 5

Fig. 6

Fig. 7

Fig. 8

Fig. 9

Fig. 10

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HSIAO-MING LU.** A potential method for in vivo range verification in proton therapy treatment. *Phys. Med. Biol.,* 2008, vol. 53, 1413-1424

- *Phys. Med. Biol.,* 2008, vol. 53, 1413-1424